# EUROPEAN PATENT SPECIFICATION

(11) **EP 2 497 486 B1**
(45) Date of publication and mention of the grant of the patent: **07.08.2013**
(21) Application number: 12170750.9
(22) Date of filing: 01.07.2004
(51) Int. Cl.: A61K 38/46

(54) **Method for treating diseases caused by fungi associated with changes of qualitative and/quantitative composition of blood extracellular DNA**
VERFAHREN ZUR BEHANDLUNG VON KRANKHEITEN VERURSACHT VON PILZEN IM ZUSAMMENHANG MIT VERÄNDERUNGEN DER QUALITATIVEN UND/ODER QUANTITATIVEN ZUSAMMENSETZUNG VON EXTRAZELLULÄRER DNA IM BLUT
MéTHODE DE TRAITEMENT DE MALADIES CAUSÉES PAR LES CHAMPIGNONS S'ACCOMPAGNANT D'ALTERATIONS DE LA COMPOSITION QUALITATIVE ET/OU& xA;QUANTITATIVE DE L'ADN EXTRACELLULAIRE DU SANG

(30) Priority: 14.07.2003 WO PCT/RU03/00304; 12.03.2004 RU 2004108057
(43) Date of publication of application: 12.09.2012
(62) Divisional of application: 04775224.1
(73) Proprietor: CLS Therapeutics Limited, Guernsey GY1 3DR (GB)
(72) Inventor: Genkin, Dmitry Dmitrievich, 197110 St. Petersburg (RU); Tets, Victor Veniaminovich, 196066 St. Petersburg (RU); Tets, Georgy Victorovich, 191040 St. Petersburg (RU)
(74) Representative: Gulde Hengelhaupt Ziebig & Schneider

(56) References cited:
- WO-A1-97/28266
- DE-A1- 4 024 530
- SUGIHARA S ET AL: "Deoxyribonuclease treatment prevents blood-borne liver metastasis of cutaneously transplanted tumour cells in mice", BRITISH JOURNAL OF CANCER, NATURE PUBLISHING GROUP, LONDON, GB, vol. 67, no. 1, 1 January 1993 (1993-01-01), pages 66-70, XP008086562, ISSN: 0007-0920
- DAVIS J C JR ET AL: "Recombinant human Dnase I (rhDNase) in patients with lupus nephritis", LUPUS, BASINGSTOKE, GB, vol. 8, no. 1, 1 January 1999 (1999-01-01) , pages 68-76, XP009124143, ISSN: 0961-2033, DOI: 10.1191/096120399678847380

## Description

### Technical field

The invention reveals to medicine and veterinary and refers to DNAse enzyme which is used for treatment of diseases caused by fungi and protozoa.

### Background art

The main method of therapy of diseases caused by bacteria, fungi and protozoa are antibiotics and chemotherapy (see Merck Manual of Diagnosis and Therapy; 16th Edition). The main way of atherosclerosis' drug therapy is therapy with statins' group's compounds inhibiting the cholesterol synthesis (see New Concepts and Paradigms in Cardiovascular Medicine: The Noninvasive Management of Coronary Artery Disease, K. Lance Gould, THE AMERICAN JOURNAL OF MEDICINE, Volume 104, June 22, 1998,2s-17s.)

Therapy of diabetes mellitus consists from three main approaches - insulin therapy, drugs increasing insulin' secretion by pancreas, drugs increasing sensitivity of tissues to the insulin or the same increasing glucose' utilization by tissues (Pharmacological Management of Diabetes: Recent Progress and Future Perspective in Daily Drug Treatment, Gérard Emilien et.al., Pharmacol, Ther. Vol. 81, No. 1, pp. 37-51, 1999).Therapy of type IV hypersensitivity is based on immunosuppressive and immunomodulating therapy (see Therapeutic Immunosupression, ed.A.W.Thomson, Ser.Immunoiogy and Medicine vol.29, Kluwer Acad.Publishers, Dordrecht, 2001).

The diseases caused by mutations in somatic genes and accompanied by somatic mosaicism development have got no etiological therapy, see Youssoufian H, Pyeritz RE. Mechanisms and Consequences of Somatic Mosaicism in Humans,. Nature Reviews Genetics, 2002;3:748-758.

The drug resistance is considered as the main problems of the antibiotic therapy of bacterial infection. The circulation of antibioticresistant strains and appearance of new ones in the process of the treatment (for example as a result of biofilms' formation in the patient's organism) are the main cause of therapy's inefficiency (The use and resistance to antibiotics in the community. Cizman M, Int J Antimicrob Agents, 2003, Apr 21: pp.297-307).

At the present time it is universally recognized that problem of antibiotic resistance has a character of global threat (Mechanisms of antimicrobial resistance: their clinical relevance in the new millennium. Sefton A.M., Drugs, 2002, vol. 62: 557-66) and it asks for development of new original antibiotics and new method with non-antibiotic mechanism of effect on the infectious process. For example vancomycin is used for infectious' treatment caused be Gram positive cocci resistant to penicillin and cephalosporin. Main disadvantages of Vancomycin are increasing of circulating number of Vancomycin-resistant strains; high toxicity; relatively narrow spectrum of activity (The threat of vancomycin resistance. PerlTM, Am J Med 1999 May 106:26S-37S).

The aforesaid data indicate that development new effective, low toxic, method demonstrated broad-spectrum activity against all species bacteria including antibiotic -resistant strains is still an extremely important task. Problems of antibiotic therapy and chemotherapy of infectious caused by fungi and protozoa are close to those of bacterial infection treatment; for example, when an established drug - amphotericin is used (Antifungal drug resistance to azoles and polyenes, Mar Masiá Canuto et.al., The Lancet Infectious Diseases, Volume 2, Issue 9, 1 September 2002, Pages 550-563; A systematic review of the antifungal effectiveness and tolerability of amphotericin B formulations, Jane P. Barrett et.al., Clinical Therapeutics,Volume 25, Issue 5, May 2003, Pages 1295-1320).

Atherosclerosis is a systemic disease that is accompanied by formation of specific atherosclerotic plaques in large and middle sized artery walls. Depends on the localization, stage and size of atherosclerotic plaques the disease has different clinical signs (angina, stroke and so on). The signs especially associated with organ dysfunction caused by systemic atherosclerosis are cured by drug therapy or by surgical operation. There is no cure for Aterosclerosis by drug therapy methods as for any systemic disease. An established method of prevention that delays the disease progression is therapy with inhibitors of 3 3-hydroxy-3-methylglutaryl-CoA (HMG CoA) reductase (Lovastatin, Parvastatine e.t.c.) leading to the inhibition of endogenous cholesterol synthesis and increasing of clearance of low density lipoproteins of blood plasma and it attenuates atherosclerosis development (New Concepts and Paradigms in Cardiovascular Medicine: The Noninvasive Management of Coronary Artery Disease, K. Lance Gould, THE AMERICAN JOURNAL OF MEDICINE, Volume 104, June 22, 1998, 2s-17s). Disadvantages of such treatment are adverse effects (A safety look at currently available statins, Moghadasian MH, Expert Opin Drug Saf 2002 Sep 1:pp.269-74) and limited efficacy (Statins: balancing benefits, efficacy and safety., Clearfield MB, Expert Opin Pharmacother, 2002, May 3:pp.469-77).

The main cause of disablement and death of patients with diabetes mellitus type 1 and 2 are complications associated with microangiopathy and macroangiopathy development. It is considered that effective metabolic control of glucose level (maintenance of glucose level and glycated hemoglobin level within the normal limits) prevents development of complications. The insulin therapy including intensive insulin therapy is the method of choice when it is impossible to reach metabolic control with other drugs (Outpatient insulin therapy in type 1 and type 2 diabetes mellitus: scientific review, DeWitt DE, Hirsch IB,JAMA, 2003, May 289:pp.2254-64).

But even if high-dose insulin therapy are used the risk of developing the complications including fatal ones is still sufficiently high (Cause-specific mortality in a population with diabetes: South Tees Diabetes Mortality Study, Roper NA, et al, Diabetes Care, 2002, Jan 25:pp.43-8). In accordance with above-mentioned the task of development of new methods of type I and type II diabetes mellitus therapy including the complication prevention methods is still topical and generally recognized.

One of the established clinical methods of treatment of r delayed-type hypersensitivity reactionsis administration of Cyclosporine A peptide (Therapeutic Immunosupression, ed. A.W.Thomson, Ser. Immunology and Medicine vol.29, Kluwer Acad. Publishers, Dordrecht, 2001). The well - known drawbacks of this method are severe adverse effects namely nephrotoxicity, hypertension and high risk of infections' development (Cyclosporine: mechanisms of action and toxicity., Graham RM, Cleve Clin J Med, 1994, Jul-Aug 61:pp.308-13). Another problem is loss the efficacy during long-term treatment, that reveals itself in the increasing risk for transplant rejection (Renal transplantation, past, present and future, Ponticelli C, et al, J Nephrol, 1999, Jul-Aug 12 Suppl 2:S105-10).Thus for treatment accompanied by qualitative and/or quantitative changes of blood extracellular DNA the wide spectra of different method are used that have got similar disadvantages: toxicity, adverse effects; low efficacy of therapy. At the same time in the real clinical practice these diseases often accompany each other. For example the therapy delayed-type hypersensitivity reactions with immunosuppressive drugs multiplies the risk of infectious diseases (Recent advances in the diagnosis and management of infection in the organ transplant recipient. Tolkoff-Rubin NE, Rubin RH; Semin Nephrol 2000 Mar 20:148-63.); atherosclerosis is a very common complication of diabetes mellitus (Diabetes and atherosclerosis: epidemiology, pathophysiology, and management, Beckman JA, Creager MA, Libby P;JAMA 2002 May 287:2570-81) and is often accompanied by systemic infectious process (Infection and atherosclerosis: potential roles of pathogen burden and molecular mimicry., Epstein SE, Zhu J, Burnett MS, Zhou YF, Vercellotti G, Hajjar D, Arterioscler Thromb Vasc Biol 2000 Jun 20:1417-20.); a number of diabetes types develops as a result of delayed-type hypersensitivity reaction (Evidence of islet cell autoimmunity in elderly patients with type 2 diabetes., Pietropaolo M, Barinas-Mitchell E, Pietropaolo SL, Kuller LH, Trucco M, Diabetes 2000 Jan 49:32-8), or during the infectious process (Systemic diseases caused by oral infection. Li X, Kolltveit KM, Tronstad L, Olsen I, Clin Microbiol Rev 2000 Oct 13:547-58 ), and leads to the high risk of infections' development (Diabetes and the risk of infection-related mortality in the U.S.,Bertoni AG, Saydah S, Brancati FL, Diabetes Care 2001 Jun 24:6 1044-9).

US 6,391,607 B1 relates to amino acid sequence variants of human DNase I that have increased DNA-hydrolytic activity. It provides nucleic acid sequences encoding such hyperactive variants, thereby enabling the production of these variants in quantities sufficient for clinical use. Further it relates to pharmaceutical compositions and therapeutic uses of hyperactive variants of human DNase I.

According to DE 40 24 530 A1 viruses and viral diseases are controlled in humans and animals by treatment with nucleases (I) isolated from bovine pancreas. (I) also include ribonucleases and deoxyribonucleases, specifically DNase I.

SUGIHARA S ET AL: describe in BRITISH JOURNAL OF CANCER, NATURE PUBLISHING GROUP, LONDON, GB, vol. 67, no. 1, 1 January 1993 (1993-01-01), pages 66-70, XP008086562 ISSN: 0007-0920 that a deoxyribonuclease treatment prevents blood-borne liver metastasis of cutaneously transplanted tumour cells in mice.

DAVIS J C JR ET AL: describe in LUPUS, BASINGSTOKE, GB, vol. 8, no. 1, 1 January 1999 (1999-01-01), pages 68-76, XP009124143 ISSN: 0961-2033 the use of recombinant human Dnase I (rhDNase) in patients with lupus nephritis.

### Disclosure of the invention

The solving of the aim of development of high-performance and low-toxic method of treatment of the diseases that are accompanied by quantitative and/or qualitative change of composition of blood plasma extracellular DNA being diseases caused by fungi and protozoa is the basis of this invention.

According to the invention this task is resolved by introducing of blood extracellular DNA destroying agent into a systemic blood circulation for treating diseases caused by fungi and protozoa. As the agent destroying blood extracellular DNA DNase enzyme can be introduced in systemic circulation: enzyme DNAase can be introduced in systemic circulation in doses that provide change of electroforetic profile of blood extracellular DNA that can be detected by puls-gel-electroforesis, whereby the DNase enzyme can be administrated at doses and regimens that can provide blood DNA-hydrolytic level measured in blood plasma and exceeded 150 Kunitz units per liter of plasma, and this level can be supported for more than 12 hours during 24 hours in total.

Development of diseases caused by fungi and protozoa is accompanied by quantitative and/or qualitative change of blood extracellular DNA, but in source of available data, there are no knowledge about genetic repertoire of extracellular blood DNA of patients with above-mentioned diseases, about biological role of extracellular blood DNA in these diseases and about potential therapeutic effect of blood extracellular DNA destroying with the purpose of treatment of these diseases; so, taking into account all aforesaid, the invention conformances to requirements of "novelty" criteria (N).

As the applicant established, the extracellular blood DNA of patients with foregoing diseases contains the unique quantitative and qualitative repertoire of genes and regulating genetic elements which great differ from the repertoire of DNA which is described in human genome. In contrast to inracellular DNA the extracellular DNA of these patients contains mainly unique human genes. Extracellular bacterial and fungal DNA was found out in biofilms' matrix and blood plasma of the infected human.

It was established that blood extracellular DNA including extracellular DNA of bacteria, fungi and protozoa promotes the development of diseases listed above.

It was established that destruction of blood extracellular plasma DNA leads to therapeutic effect on the diseases listed above.

Aforesaid new characteristics of the claimed invention are based on new ideas about mechanisms of the described diseases. In this way the claimed method conformances to requirements of "invention step" criteria (IS).

### Brief description of the drawings

As set for below the invention has been explained by detailed description of embodiments without references to drawings.

### Preferred embodiment

The claimed inventive method is realized by following:

### Materials and methods.

The following agents that destroy extracellular blood DNA were used: bovine pancreatic DNase (Sigma and Samson-Med), recombinant human DNase I (Gentech), DNA-hydrolyzing anti-DNA antibodies isolated from the blood of patients with lupus erythematosus according to Shuster A.M. (Shuster A.M. et.al., Science, v. 256,1992, pp.665-667).

Extracellular DNA from blood plasma was isolated as follows: fresh plasma with (no more than 3-4 hours after sampling) with an added anticoagulant (sodium citrate) was centrifuged on Ficoll-PlaquePlus (Amersham-Pharmacia) during 20 minutes at 1500 g at room temperature. ½ of plasma was detached, not affecting the rest of cells on the Ficoll pillow, and further centrifuged at 10000 g during 30 min for separation from cell fragments and debris. Supernatant was detached, without affecting of the sediment, and was toped up to 1% of sarkosil, 50MM tris-HCl, pH 7,6, 20 MM EDTA, 400 MM NaCl, and than mixed with equal volume of phenol-chloroform(1:1) mixture. The prepared emulsion was incubated during 2 hours at t=65°C, then phenol-chloroform mixture was separated by centrifuging (500g during 20 minutes, room temperature).

The procedure of deproteinization with phenol - chloroform mixture was repeated 3 times, and then the water phase was processed with chloroform and diethyl ether. Separation from organic solvents was made by centrifugation at 5000g during 15 minutes). Then equal volume of isopropanol was added to resulting aqueous phase and the mixture was incubated overnight at 0°C. After sedimentation the nucleic acids were separated by centrifugation at 10000g during 30 minutes. The sediment of nucleic acids was dissolved in of 10MM tris-HCl buffer, pH 7, 6 with 5 MM EDTA, and inflicted to the CsCl gradient (1M, 2.5M, 5.7M) in test-tube for rotor SW60Ti. The volume of DNA solution was 2 ml, volume of each step of CsCl was 1 ml. Ultracentrifugation was conducted in L80-80 (Beckman) centrifuge during 3 hours at 250000g. DNA was collected from the surface of each gradient step into fractions. These fractions were dialyzed during 12 hours (t=4°C). Presence of DNA in fractions was determined by agar electrophoresis and DNA was visualized by ethidium bromide staining. The amount of DNA was determined with spectrophotometer (Beckman DU70) in cuvet (100 mcl) at wavelength of 220-230 nm.

### Example 1.

### Treatment of the experimental sepsis caused by Candida Albicans

Group 3 - 10 mice. Clinical isolate of Candida Albicans at LD 50 dose was intravenously administered. Recombinant Dornase alpha (Genentech) was intraperitoneally administered at 1 mg/kg dose twice a day on day 2, day 3 and day 4 after contamination.

Group 4 - 10 mice. Clinical isolate of Candida Albicans at LD 50 dose was intravenously administered. Amphotericin B was intraperitoneally administered. At 20 mg/kg dose twice a day on day 2, day 3 and day 4 after contamination.

Group 5 - 10 mice. Clinical isolate of Candida Albicans was intravenously administered at LD 50 dose. Phosphate buffer was intraperitoneally administered as negative control twice a day on day 2, day 3 and day 4 contamination.

Viability of mice and their weight was estimated on the 7th day after contamination. Results are presented in the table 2.

**Table 2**

| | The mice viability at different time periods following contamination | | | |
|---|---|---|---|---|
| | 1 day | 3 day | 5 day | 7 day |
| Group 3 | 100% | 100% | 100% | 100% |
| Group 4 | 100% | 100% | 100% | 100% |
| Group 5 | 100% | 80% | 50% | 50% |

Weight of mice from group 4 on the 7^{th} day of experiment was 20% less than in group 3. This fact indicates that amfotericin B is more toxic than dornase alpha, though their protective efficacies are equal.

Thus blood extracellular DNA of the infected animals possesses negative influence on the development of the infectious process and according to the claimed method its destruction is effective in the treatment of fungi infections.

### Industrial applicability

For the realization the methods there were used well-known materials and equipment manufactured in plant conditions and according to aforesaid the invention conformances to requirements of "industrial applicability" criteria (IA).

## Claims

1. DNAse enzyme for use in treating diseases caused by fungi associated with changes of qualitative and/or quantitative composition of blood extracellular DNA, wherein the DNAse is introduced into the systemic blood circulation.

2. DNAse enzyme according to claim 1 wherein the DNAase enzyme is introduced in doses sufficient to provide blood extracellular DNA electrophoretic profile change, wherein the DNAase enzyme is introduced in doses and regimens which provide blood plasma DNA-hydrolytic activity measured in blood plasma, to exceed 150 Kunitz units per litre of plasma during more than 12 hours in total within 24 hours.

## Patentansprüche

1. DNase-Enzym zur Verwendung bei der Behandlung von Krankheiten verursacht von Pilzen, die mit Änderungen der qualitativen und/oder quantitativen Zusammensetzung von extrazellulärer DNA im Blut verbunden sind, wobei die DNase in den systemischen Blutkreislauf eingebracht wird.

2. DNase-Enzym nach Anspruch 1, wobei das DNase-Enzym in hinreichenden Dosen eingebracht wird, um eine Änderung des elektrophoretischen Profils extrazellulärer DNA im Blut zu ergeben, wobei das DNase-Enzym in Dosen und Regimes eingebracht wird, die eine im Blutplasma gemessene Blutplasma-DNA-Hydrolyseaktivität von mehr als 150 Kunitz-Einheiten pro Liter Plasma im Laufe von insgesamt mehr als 12 Stunden innerhalb 24 Stunden ergeben.

## Revendications

1. Enzyme DNase destinée à une utilisation dans le traitement de maladies causées par les champignons associée à des changements de composition qualitative et/ou quantitative d'ADN extracellulaire dans le sang, dans laquelle la DNase est introduite dans la circulation sanguine systémique.

2. Enzyme DNase selon la revendication 1, moyennant quoi l'enzyme DNase est introduite dans des doses suffisantes pour modifier le profil électrophorétique d'ADN extracellulaire dans le sang et l'enzyme DNase est introduite dans des doses et des schémas posologiques qui procurent une activité hydrolytique d'ADN de plasma sanguin, mesurée dans le plasma sanguin, pour dépasser 150 unités Kunitz par litre de sang pendant plus de 12 heures au total dans un délai de 24 heures.
